# EUROPEAN PATENT APPLICATION

(11) **EP 0 945 137 A1**
(43) Date of publication of application: **29.09.1999**
(21) Application number: 99250005.8
(22) Date of filing: 11.01.1999
(51) Int. Cl.: A61K 47/34, A61K 9/70

(54) **Sustained-release pharmaceutical composition**

(30) Priority: 13.01.1998 JP 464098
(71) Applicant: Meiji Seika Kaisha, Ltd., Chuo-ku, Tokyo 104-0031 (JP); Taisei Corporation, Tokyo 163-0606 (JP)
(72) Inventor: Ueno, Shinobu, c/o Meiji Seika Kaisha Ltd., Odawara-shi, Kanagawa 250-0852 (JP); Kawashima, Junichi, c/o Meiji Seika Kaisha Ltd., Odawara-shi, Kanagawa 250-0852 (JP); Iinuma, Katsuharu, c/o Meiji Seika Kaisha Ltd., Odawara-shi, Kanagawa 250-0852 (JP); Tomosawa, Takashi, c/o Taisei Corporation, Tokyo 163-0606 (JP); Saito, Yuji, c/o Taisei Corporation, Tokyo 163-0606 (JP); Shibayama, Masako, c/o Taisei Corporation, Tokyo 163-0606 (JP)
(74) Representative: Ziebig, Marlene, Dr. Dipl.-Chem.

(57) **Abstract**

The present invention relates to a sustained-release pharmaceutical composition comprising a hydroxyalkanoic acid copolymer consisting of a 3-hydroxybutyric acid unit and a 4-hydroxybutyric acid unit, and a biologically active substance. The sustained-release pharmaceutical composition of the present invention can regulate the degree of release of a biologically active agent easily by changing the ratio of the 3-hydroxybutyric acid (3HB) component to the 4-hydroxybutyric acid (4HB) component in P(3HB-co-4HB) as a basic substance and by changing the thickness of its sheet.

## Description

### FIELD OF THE INVENTION

The present invention relates to a sustained-release pharmaceutical composition comprising a biologically active substance and an in vivo degradable high-molecular compound wherein the release of the biologically active substance is controlled.

### BACKGROUND OF THE INVENTION

A variety of sustained-release pharmaceutical compositions have been developed heretofore for drugs requiring continuous administration. According to these sustained-release pharmaceutical compositions, a therapeutically effective amount of a drug is released for a longer time than usual pharmaceutical preparations, thus effectively bringing out the potential effectiveness of the drug while reducing the frequency of administration to prevent the disappearance of efficacy and the worsening of symptoms in case the patient forgets to take the drug. In recent years, drugs can be released at constant rate particularly as sustained-release pharmaceutical compositions, and proposals have been made for a wide variety of sustained-release pharmaceutical compositions where the rates of release of drugs are substantially zero-order.

A wide variety of sustained-release pharmaceutical compositions comprising in vivo degradable polymeric substances and biologically active substances have been reported, and the in vivo degradable polymeric substances include e.g. polylactic acid, a lactic acid-glycolic acid copolymer, and a hydroxybutyric acid-glycolic acid copolymer (Japanese Patent Publication No. 57087/1989, WO94/10982, Japanese Laid-Open Patent Publication Nos. 151,322/1996 and 217,691/1996). Further, it is known that a 3-hydroxybutyric acid polymer (referred to hereinafter as P(3HB)) is also used in microcapsules for regulatory peptides where the release of the active ingredient is controlled, or in microspheres containing Lasted (Japanese Laid-Open Patent Publication No. 431,119/1986, Drug Delivery System, 7(5), 367-371, 1992, and 8(2), 131-136, 1993).

However, these sustained-release pharmaceutical compositions suffer from the problems that their industrial production is difficult owing to complicated compositions and the control of release of biologically active substances is difficult.

An in vivo degradable polymeric substance is a polymeric substance which can be decomposed by microorganisms. One example is a hydroxyalkanoic acid copolymer (referred to hereinafter as P(3HB-co-4HB)) consisting of a 3-hydroxybutyric acid unit (referred to hereinafter as 3HB component) and a 4-hydroxybutyric acid unit (referred to hereinafter as 4HB component), and this copolymer exhibits various properties depending on the composition of the copolymer, so it attracts considerable attention as a biodegradable plastic material. However, there is no report on a sustained-release pharmaceutical composition using the hydroxyalkanoic acid copolymer.

### SUMMARY OF THE INVENTION

The object of the present invention is to provide a sustained-release pharmaceutical composition wherein the release of a biologically active substance can be easily controlled.

As a result of their earnest study to arrive at the present invention, the present inventors found that an in vivo degradable polymeric substance is useful as a sustained-release base.

The in vivo degradable polymeric compound includes a hydroxyalkanoic acid copolymer (referred to hereinafter as P(3HB-co-4HB)) consisting of a 3-hydroxybutyric acid unit and a 4-hydroxybutyric acid unit, and the ratio of the 3-hydroxybutyric acid (3HB) component to the 4-hydroxybutyric acid (4HB) component in the P(3HB-co-4HB) is varied whereby the releasability of a biologically active substance can be controlled, and the releasability of a biologically active substance can also be controlled according to thickness during formation. Accordingly, this sustained-release pharmaceutical composition is advantageous over conventional sustained-release pharmaceutical compositions in respect to ease of control in releasing a biologically active substance.

The present invention is a sustained-release pharmaceutical composition comprising a hydroxyalkanoic acid copolymer consisting of a 3-hydroxybutyric acid unit and a 4-hydroxybutyric acid unit, and a biologically active substance.

The ratio of the 3-hydroxybutyric acid component : 4-hydroxybutyric acid component in the hydroxyalkanoic acid copolymer is in the range of 1 : 99 to 90 : 10, preferably 10 : 90 to 40 : 60.

The sustained-release pharmaceutical preparation includes a formed sheet of 0.01 to 0.5 mm in thickness.

The biologically active substance is one or more members selected from the group consisting of an antibiotic, an anti-fungus agent, an anti-hyperlipemia agent, an agent for circulatory organs, an anti-platelet agent (anti-platelet coagulation inhibitor), an anti-tumor agent, an antipyretic, lenitive, an anti-inflammatory agent, a cough medicine and an expectorant, a sedative, an anti-epilepsy agent, an anti-ulcer agent, an anti-depression agent, an anti-allergy agent, a cardiotonic, a therapeutic agent for arrhythmia, a vasodilator drug, an antihypertensive diuretic, a therapeutic agent for diabetes, a hormone agent and a bone absorption inhibitor.

### DETAILED DESCRIPTION OF THE INVENTION

The P(3HB-co-4HB) used in the present invention may be obtained from any microorganisms having the ability to produce P(3HB-co-4HB). Examples are microorganisms belonging to the genera Comamonas, Alcaligenes and Rhodococcus and having the ability to produce P(3HB-co-4HB).

Specifically, the microorganisms include Comamonas acidovorans, Alcaligenes eutrophus, Alcaligenes latus, and strains belonging to the genus Rhodococcus. Easily obtained strains include Comamonas acidovorans IFO 13582, Alcaligenes eutrophus ATCC 17699, Alcaligenes eutrophus ATCC 11599, Alcaligenes latus ATCC 29713, Rhodococcus sp. NCIMB 40126, and Rhodococcus sp. ATCC 19070.

To permit P(3HB-co-4HB) to be accumulated in the microorganism, the microorganism is inoculated into a suitable medium selected depending on the type of the microorganism, and grown by culturing in a usual manner. The medium may be any medium generally used for culturing the microorganism, and if a microorganism belonging to the genus Comamonas is used, 3-hydroxybutyric acid and 4-hydroxybutyric acid are used as the carbon source. As other carbon sources, mention is made of e.g. alkane diols containing even-numbered carbon atoms, γ-butyrolactone, 4-aminobutyric acid etc. Other culture conditions such as medium pH, culture temperature, culture time etc. can be suitably determined depending on the type of the microorganism.

By selecting these conditions, for example by changing the proportion of carbon sources in the medium, P(3HB-co-4HB) containing the 3HB and 4HB components a varying ratio can be obtained (Japanese Laid-Open Patent Publication No. 336,982/1993 and Japanese Patent Application No. 193,935/1996).

In the present invention, P(3HB-co-4HB) accumulated in the microorganism having the ability to produce P(3HB-co-4HB) is extracted by directly adding an organic solvent to the wet microorganism separated from the culture by centrifugation or filtration and then sufficiently mixing them. In adding an organic solvent in the above extraction step, it is more preferable that a hydrophilic organic solvent is added as a first organic solvent and mixed well, followed by addition of a second organic solvent for further extraction of P(3HB-co-4HB).

In this case, direct extraction from the wet microorganism with the organic solvent suffices thus making it unnecessary to dry the microorganism, so this method can be superior economically and in productivity. Furthermore, according to the present invention, P(3HB-co-4HB) containing 4HB at a relatively high ratio can be selectively extracted owing to the difference in affinity between 4HB and 3HB in P(3HB-co-4HB) for the second organic solvent.

In the present invention, the first organic solvent added to the wet microorganism may be any hydrophilic solvent such as methanol, ethanol, acetone, acetonitrile, tetrahydrofurane, N,N-dimethylformamide, N,N-dimethylacetamide, 1-propanol, 2-propanol, dimethylsulfoxide, 1,3-dioxane, 1,4-dioxane or the like, among which methanol, ethanol, acetonitrile and acetone are preferable and methanol is more preferable.

The second organic solvent may be any organic solvent in which P(3HB-co-4HB) can be dissolved, and examples are acetone, ethyl acetate, acetonitrile, tetrahydrofurane, 2-butanone, chloroform, dichloromethane and dichloroethane, among which acetone, ethyl acetate, acetonitrile, tetrahydrofurane and 2-butanone are preferable.

The amount of the first organic solvent is in the range of 100 ml to 1 L relative to 100 g of the wet microorganism, and the amount of the second organic solvent is in the range of 400 ml to 3 L relative to 100 g of the wet microorganism.

The time after addition of the first organic solvent until addition of the second organic solvent is not limited insofar as the first organic solvent sufficiently permeates the microorganism, and usually 1 to 10 hours are preferable. The extraction time after addition of the second organic solvent is usually preferably 1 to 48 hours. Although the temperature in the mixing step is not particularly limited, it is preferably that it not be higher than the boiling point of the organic solvent added.

After the P(3HB-co-4HB) is extracted from the microorganism, the P(3HB-co-4HB) can be recovered in any method known in the art. Specifically, the microorganism in the organic solvent is filtered or centrifuged to remove microbial residues, and the extract thus obtained is mixed with a poor solvent whereby the P(3HB-co-4HB) can be recovered as precipitates. The type of poor solvent is not particularly limited, and examples are methanol, hexane, pentane, water etc., preferably methanol and hexane.

The P(3HB-co-4HB) becomes ductile as the ratio of the 4HB component is increased, and it further becomes elastic when the ratio of the 4HB component exceeds 60 %. Because its crystallinity is decreased as the ratio of the 4HB component in the copolymer is increased, materials with various physical properties can be obtained by changing their composition. It is easy to handle copolymers containing preferably 10 to 99 % 4HB component, more preferably 60 to 90 % 4HB component. Further, the degree of release of a drug can be regulated according to the ratio of the 4HB component, and it was revealed that sustained efficacy is exhibited as the ratio of the 4HB component is increased.

The sustained-release pharmaceutical composition of the present invention is obtained by dissolving P(3HB-co-4HB) and a biologically active substance in a suitable solvent, stirring them to prepare an uniform solution and forming it into various forms of pharmaceutical preparations. As necessary, suitable additives (surfactants, plasticizers, lipids etc.) can be added.

The solvent is not particularly limited insofar as P(3HB-co-4HB) can be dissolved therein, and examples are 2-methyl propane, chloroform, acetone, ethyl acetate etc. and these may be used alone or in combination thereof, and 2-methyl propane is particularly preferable.

The type of the physiologically acceptable substance used in the present invention is not particularly limited, and use is made of one or more members selected from the group consisting of an antibiotic, an anti-fungus agent, an anti-hyperlipemia agent, an agent for circulatory organs, an anti-platelet agent (anti-platelet coagulation inhibitor), an anti-tumor agent, an antipyretic, a lenitive, an anti-inflammatory agent, a cough medicine and an expectorant, a sedative, an anti-epilepsy agent, an anti-ulcer agent, an anti-depression agent, an anti-allergy agent, a cardiotonic, a therapeutic agent for arrhythmia, a vasodilator drug, an antihypertensive diuretic, a therapeutic agent for diabetes, a hormone agent and a bone absorption inhibitor. However, these physiologically acceptable substances are preferably substances dissolved in the solvent.

The content of a biologically active substance is about 0.01 to 2 %, preferably 0.01 to 0.02 % of P(3HB-co-4HB).

The concentration of P(3HB-co-4HB) in the solvent is varied depending on the thickness of a desired sheet in the range of a high concentration for a thick sheet to a low concentration for a thin sheet, and the concentration is usually 1 to 5 %, preferably 1.5 to 12 %. A concentration of lower than 1 % does not give uniform thickness, while a concentration of more than 15 % fails to attain uniform concentration so the desired effect cannot be achieved.

P(3HB-co-4HB) and a biologically active substance are dissolved in the solvent, stirred to prepare an uniform solution, and formed into various forms of pharmaceutical preparations. The forms of pharmaceutical preparations include, but are not limited to, granulates, capsules, tablets, microspheres etc., preferably sheets. In the method of forming a sheet, the above mixed solution is pipetted into a flat-bottom vessel of predetermined volume and left at room temperature until its crystallization becomes saturated. The solvent is evaporated whereby a sheet-shaped release control system can be obtained.

In the release control system of the present invention, the elution of a biologically active substance is varied depending on the thickness of the formed sheet. That is, its elution is rapid with a thickness of 0.04 mm and slow with a thickness of 0.3 mm. Accordingly, the efficacy can be prolonged by this combination of thickness.

### [Production Example]

One L medium (pH 7.0) consisting of polypeptone (10g), yeast extract (5 g) and sodium chloride (5 g) was added to each of 3 jar fermentors and sterilized. Ten ml of a culture of a cryopreserved microorganism (Comamonas acidovorans IFO 13582) was inoculated aseptically into each jar fermentor and cultured at 30 °C under aeration. The pH of the culture was controlled always within pH 6.8 to 7.2 with 0.1 % sodium hydroxide. The cells grown after 24 hours were recovered by centrifugation, and the medium was exchanged by a nitrogen-limited medium containing 3 mixed carbon sources and sodium 3-hydroxybutyrate at ratios shown below, and the microorganism was cultured under aeration at 30 °C for 48 hours.

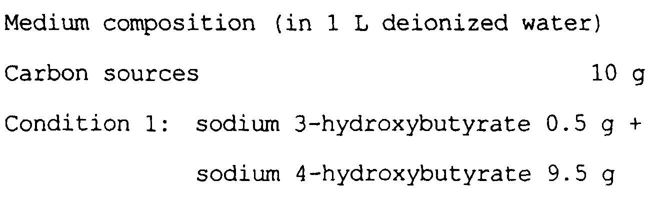

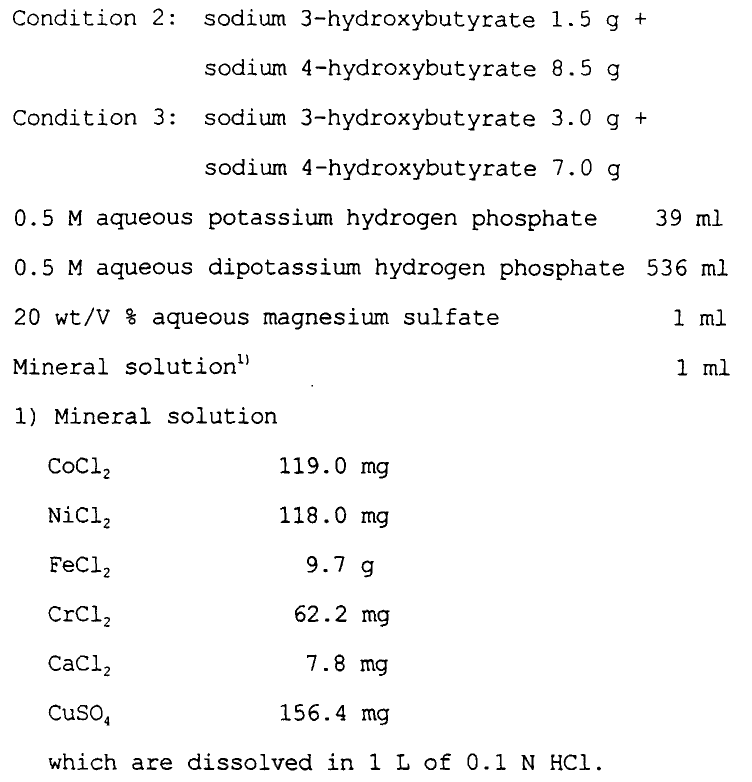

After culturing, the microorganism was recovered and lyophilized. Polyester was extracted from the dried microorganism by a soxhlet extractor (extracting solvent: chloroform). The extract was filtered and concentrated in an evaporator and added to hexane whereby the polyester was precipitated. The polyester content was calculated from the ratio of the weight of the dried polyester to the weight of the microorganism after extraction. The composition of the polyester was determined by gas chromatography and ¹H-NMR spectrum.

The results are shown in Table 1. The microorganism can produce P(3HP-co-4HB) having a different composition by changing the compounding ratio of sodium 3-hydroxybutyrate and sodium 4-hydroxybutyrate as carbon sources.

**Table 1**

| Condition | Polyester Content (weight-%) | Copolymer Composition (mol-%) | |
|---|---|---|---|
| | | 3HB | 4HB |
| 1 | 23 | 10 | 90 |
| 2 | 26 | 31 | 69 |
| 3 | 27 | 59 | 41 |

### EXAMPLES

Hereinafter, the present invention is described in more detail by reference to Examples. However, the technical scope of the present invention is not limited to the Examples.

### Example 1

P(3HB-co-4HB) in the amount of 0.4 g, 1.6 g or 2.8 g having a 4HB content of 90 % was dissolved in 25 ml dimethylethane, and 0.005 g of acetaminophen was added to each solution and completely dissolved. Then, each solution was introduced into a flat vessel of 9 cm in diameter and left in a horizontal draft until its crystallization became saturated, to prepare a release control system (sheet).

The resulting sheets were 0.04 mm, 0.18 mm and 0.32 mm in thickness respectively and these sheets were examined in a releasability test in an acidic solution at pH 1.2. In the test, each sheet was shaken and stirred at 50 rpm in a thermostatic chamber at 37 °C and an aliquot was taken at suitable intervals to monitor the maximum absorbance of acetaminophen at 240 nm. The results of the releasability test are shown in Table 2 below.

**Table 2**

| Degree of release of acetaminophen (%) | | | | |
|---|---|---|---|---|
| sheet thickness | 1 hour | 2 hours | 3 hours | 6 hours |
| 0.04 mm | 91.3 | 98.2 | 100 | 100 |
| 0.18 mm | 66.1 | 83.7 | 91.5 | 100 |
| 0.32 mm | 42.0 | 58.2 | 70.4 | 84.9 |

It is understood that the degree of release of the drug after 1 hour can be controlled in the range of 40 % to 90 %, and that the degree of release of the drug after 3 hours can be controlled in the range of 70 % to 100 %.

### Example 2

P(3HB-co-4HB) in the amount of 0.4 g having a 4HB content of 90 %, 69 % or 41 % was dissolved in 25 ml solvent, and 0.005 g of acetaminophen was added to each solution and completely dissolved. Then, the solution was introduced into a flat vessel of 9 cm in diameter and left in a horizontal draft until its crystallization became saturated, to prepare a release control system (sheet) of 0.03 mm in thickness.

As a comparative example, P(3HB) was also subjected to the same procedure to prepare a sheet of 0.03 mm in thickness.

These sheets were examined in the same releasability test as in Example 1. The results are shown in Table 3 below.

**Table 3**

| Degree of release of acetaminophen (%) | | | | |
|---|---|---|---|---|
| ratio of the units | 1 hour | 2 hours | 3 hours | 6 hours |
| P(3HB-co-90 % 4HB) | 91.7 | 91.4 | 97.5 | 98.7 |
| P(3HB-co-69 % 4HB) | 90.2 | 95.2 | 97.3 | 99.2 |
| P(3HB-co-41 % 4HB) | 67.6 | 80.7 | 87.8 | 97.1 |
| P(3HB) | 4.9 | 7.8 | 10.2 | 14.4 |

As can be seen from Table 2, the degree of release of the drug can be regulated using a sheet containing the 4HB component at a different ratio in P(3HB-co-4HB).

As described above, the sustained-release pharmaceutical composition of the present invention can regulate the degree of release of a biologically active agent easily by changing the ratio of the 3HB component to 4HB component in P(3HB-co-4HB) as a basic substance and by changing the thickness of its sheet. Further, the industrial production of the composition of the present invention is also easy.

## Claims

1. A sustained-release pharmaceutical composition comprising a hydroxyalkanoic acid copolymer consisting of a 3-hydroxybutyric acid unit and a 4-hydroxybutyric acid unit, and a biologically active substance.

2. A sustained-release pharmaceutical composition according to claim 1 wherein the ratio of the 3-hydroxybutyric acid component : 4-hydroxybutyric acid component in the hydroxyalkanoic acid copolymer is in the range of 1 : 99 to 90 : 10.

3. A sustained-release pharmaceutical composition according to claim 1 wherein the ratio of the 3-hydroxybutyric acid component : 4-hydroxybutyric acid component in the hydroxyalkanoic acid copolymer is in the range of 10 : 90 to 40 : 60.

4. A sustained-release pharmaceutical composition according to any one of claims 1 to 3, which is formed into a sheet of 0.01 to 0.5 mm in thickness.

5. A sustained-release pharmaceutical composition according to any one of claims 1 to 4, wherein the biologically active substance is one or more members selected from the group consisting of an antibiotic, an anti-fungus agent, an anti-hyperlipemia agent, an agent for circulatory organs, an anti-platelet agent (anti-platelet coagulation inhibitor), an anti-tumor agent, an antipyretic, a lenitive, an anti-inflammatory agent, a cough medicine and an expectorant, a sedative, an anti-epilepsy agent, an anti-ulcer agent, an anti-depression agent, an anti-allergy agent, a cardiotonic, a therapeutic agent for arrhythmia, a vasodilator drug, an antihypertensive diuretic, a therapeutic agent for diabetes, a hormone agent and a bone absorption inhibitor.
